# EUROPEAN PATENT APPLICATION

(11) **EP 1 380 300 A1**
(43) Date of publication of application: **14.01.2004**
(21) Application number: 02720008.8
(22) Date of filing: 25.03.2002
(51) Int. Cl.: A61K 31/565, A61K 31/215, A61P 3/10, A61P 3/06, A61P 3/08

(54) **OESTROGEN AND FATTY ACID MONOESTER AS A HYPOLIPIDAEMIC AND ANTIDIABETIC AGENT**

(30) Priority: 28.03.2001 ES 200100785
(71) Applicant: Oleoyl-Estrone Developments, S.L., 08028 Barcelona (ES)
(72) Inventor: ALEMANY LAMANA, Mariano, E-08035 Barcelona (ES); REMESAR BETLLOCH, Francisco Javier, E-08190 Sant Cugat del Valles (ES); FERNANDEZ LOPEZ, José Antonio, E-08004 Barcelona (ES)
(74) Representative: SUGRANES - VERDONCES - FERREGÜELA
(86) International application number: PCT/ES2002/000151
(87) International publication number: WO 2002/076465

(57) **Abstract**

Oleoyl estrone or estrone monooleate is a fatty-acid monoester of an estrogen which appear to be useful for the preparation of a medicament for the treatment and/or profilaxis of a human suffering from a metabolic disease selected from the group consisting of diabetes mellitus type 2, hyperlipidemia (hypertriacylglyceridemia, hyperlipoproteinemia, hypercholesterolemia) and the Metabolic Syndrome X associated with glucose intolerance, hyperinsulinemia, or insulin resistance. Compared with insulin, oleoyl-estrone has the advantage of being aministered orally. The effectiveness of oleoyl-estrone as oral antidiabetic is conspicuous since it does not act as a simple hypoglycemic agent, but goes to the root of the problem lowering insulin resistance. The additional hypolipemic effect allows to extend its use for the treatment of dislipemias.

## Description

The present invention relates to new uses of known chemical compounds for fighting against metabolic diseases in humans.

### BACKGROUND ART OF THE INVENTION

Diabetes mellitus is a widely extended pathology; it is found in two main forms: diabetes type 1 and diabetes type 2. In diabetes type 1, or juvenil diabetes, or insulin-dependent diabetes mellitus (IDDM), there is a severe lack of insulin production and release into the bloodstream. The lack of insulin is treated by periodical injections of the hormone; a failure to do so elicits a dramatic rise in blood glucose that leads to the presence of glucose in the urine and the massive passing of this urine. Diabetes type 2, or maturity-onset diabetes, or non-insulin-dependent diabetes mellitus (NIDDM) is more a consequence of the inability of the insulin present in the blood to effectively modulate the circulating glucose levels than to lack of the hormone. In NIDDM there is an "insulin resistance", the effects are not as dramatic as in IDDM and the diabetes is often combined with other physiological alterations, constituting the so-called "Metabolic Syndrome X" (sometimes referred as simply "X Syndrome" or "Metabolic Syndrome"), in which in addition to diabetes (hyperglycemia and hyperinsulinemia) the patients show other conditions, such as arterial hypertension and marked increases in blood lipids. Diabetes type 2 is more common than type 1 and is more susceptible to alternative treatment with diet and/or oral hypoglycemic drugs.

There are several antidiabetic and/or hypoglycemic agents in the market such as insulin, biguanides, sulfonylurea derivatives, thiazolidinediones, and there are others (e.g. vanadium and tungsten compounds) proposed but not marketed yet. Nevertheless diabetes mellitus is still a major global health problem which is recognized by the World Health Organization to be reaching epidemic proportions. It is now the fourth leading cause of death in most developed countries and a disease that is increasing rapidly in countries undergoing industrialization. Diabetes mellitus is a disease in which there is a defective carbohydrate metabolism and is characterized by abnormally large amounts of sugar glucose in the blood and urine. Diabetes mellitus can eventually damage the eyes, kidneys, heart, and limbs, and can endanger pregnancy. Diabetes type 2 is usually associated with other metabolic diseases such as hyperlipidemia, hypertension and the so-called Metabolic Syndrome X. Therefore, the search for new therapeutical agents to fight these conditions is an active field of research.

EP 771.817-A teaches the use of substantially pure fatty-acid monoesters of estrogens for the treatment of obesity and/or overweight, the fatty-acid being selected from oleic, linoleic, linolenic, stearic, palmitic, palmitoleic and arachidonic acids; and the estrogen being selected from estrone, diethylstilbestrol, estriol and ethinyl estradiol. Besides, this document only teaches the use of the fatty-acid monoester via iv-infusion.

### SUMMARY OF THE INVENTION

The present invention provides a new method of treatment and/or prophylaxis of a human suffering from a metabolic disease selected from the group consisting of diabetes mellitus type 2, hyperlipidemia, and the Metabolic Syndrome X associated with glucose intolerance, hyperinsulinemia, or insulin resistance, said method comprising the use of oleoyl-estrone, which is a specific fatty-acid monoester of an specific estrogen. The preparation of oleoyl-estrone or estrone monooleate (CAS RN 180003-17-2) was first described in the above-mentioned EP 771.817-A document.

As illustrated in the accompanying examples, now it has been found that oleoyl-estrone, specially when administered orally, contributes both to the lowering of blood glucose levels, and to the lowering of blood lipid levels in rats. Thus, the present invention relates to the use of oleoyl-estrone for the preparation of a medicament for the treatment and/or prophylaxis of a human suffering from a metabolic disease selected from the group consisting of diabetes mellitus type 2, hyperlipidemia, and the Metabolic Syndrome X associated with glucose intolerance, hyperinsulinemia, or insulin resistance.

In a particular embodiment of the invention, the metabolic disease is diabetes mellitus type 2 (non-insulin-dependent).

In other particular embodiment of the invention, the metabolic disease is hyperlipidemia. Within hyperlipidemia, the treatment and/or prophylaxis of hypertriacylglyceridemia, hypercholesterolemia and hyperlipoproteinemia are specially preferred.

In still other particular embodiment, the metabolic disease is Metabolic Syndrome X associated with glucose intolerance, hyperlipidemia, hyperinsulinemia, or insulin resistance.

Oleoyl-estrone can be administered through any pharmaceutically appropriate via. However, the oral administration is specially preferred. The possibility of oral administration involves an advantage over the administration of insulin, which must be injected.

As illustrated by accompanying examples, the oral administration of oleoyl-estrone to rats causes: a marked fall (towards normal levels) of plasma lipids, the normalization of glycemia, and the decrease in the insulin concentration needed to elicit these effects, with the result of the overall disappearance of the alterations that characterized the type 2 diabetes in this animal model. The effectiveness of oleoyl-estrone as oral antidiabetic is conspicuous since it does not act as a simple hypoglycemic agent, but goes to the root of the problem lowering insulin resistance. The additional hypolipemic effect allows to extend its utilization to the treatment of dislipemias.

Accompanying examples also illustrate that the eventual use of oleoyl-estrone as oral antidiabetic drug appears to have an outstanding level of safety. The easy administration and the lack of unwanted secondary effects support oleoyl-estrone as a candidate drug to be used as oral antidiabetic (type 2) with safety and effectiveness in reducing insulin resistance dislipemia and hyperlipemia.

### BRIEF DESCRIPTION OF THE DRAWINGS

Figure 1 is a lipoproteinogram of plasma from normal-weight rats (Zucker Fa/?), showing the effect of oleoyl-estrone on lipoprotein distribution. I = intact; C = control; T = treated with oleoyl-estrone; HDL = high-density lipoproteins; LDL = low-density lipoproteins; VLDL = very low-density lipoproteins.
Figure 2 is a lipoproteinogram analogous to the one of Figure 1, but using obese rats (Zucker fa/fa).
Figure 3 shows the changes in plasma lipid concentrations (CM = chylomicra; LPdp = lipoprotein-depleted plasma), expressed in grams per liter (g/L), in lean rats receiving oleoyl-estrone (OE; daily dosage of 10 µmol/kg in 0.2 ml of sunflower oil) compared with controls (C; receiving only the oil). Codes in bars are: triacylglycerols = white; phospholipids = lines; cholesterol = dots.
Figure 4 is analogous to Figure 3, but for obese rats.

### EXAMPLES

### EXAMPLE 1: Effect of oleoyl-estrone on glucose, triacylglycerols and total cholesterol

A study was carried out using the Zucker fa/fa rat as experimental animal model for type 2 diabetes. In these rats a marked insulin resistance is known; their circulating glucose levels are somewhat higher than in normal rats, but their insulin levels are much higher and show marked hypertriacylglycerolemia and hypercholesterolemia. Four groups of adult Zucker fa/fa rats were used, two groups of 6 male rats each and two groups of 6 female rats each. For each sex, the first group was used as control and the other was treated with the drug. All rats were kept under standard housing conditions, with free access to water and pellet food. The animals received a daily gavage of 0.2 ml of sunflower oil by means of a stomach tube. In the treated-rat groups, the oil contained enough oleoyl-estrone dissolved to provide a daily dose of 10 micromol per kg of rat weight. After 10 days of daily gavages, the rats were killed and their blood was recovered, being used to obtain plasma by centrifugation. Plasma samples were used for the analysis of glucose, total cholesterol and triacylglycerols by means of a dry-strip procedure (Spotchem, Menarini, Firenze, Italy), the levels of 3-hydroxybutyrate were measured with the kit 907979 from Boehringer Mannheim (Mannheim, Germany), and non-esterified fatty acids with the kit 1383175 from the same company. Insulin levels were measured through radioimmunoassay (rat insulin kit, Amersham, Amersham, United Kingdom). Results are shown in Table 1.

Analysis of the results shows that oral treatment with oleoyl-estrone results in significant decreases (P<0.05) in the plasma concentration of glucose, triacylglycerols and total cholesterol, with a marked decrease in insulin levels, from the hyperinsulinaemic condition of controls to much more "normalized" levels in treated rats. There are no changes in non-esterified fatty acids nor in 3-hydroxybutyrate, which indicates that the drop in insulin resistance observed could not be traced to changes in non-esterified fatty acid levels, since they did not change and insulin resistance decreased sharply.

### EXAMPLE 2: Dependence of the effect on the oleoyl-estrone dose

Eight groups of 6 normal Wistar female rats each were used. The animals were kept under standard housing conditions and had free access to pellet food and tap water. All the rats received a daily oral gavage of 0.2 ml of sunflower oil through a stomach tube. This oil contained different amounts of oleoyl-estrone so that the rats received daily doses of 0, 0.2, 0.5, 1, 2, 5, 10 or 20 micromols per kg of body weight. After 10 days of daily gavages, the rats were killed and their blood extracted and used to obtain plasma through centrifugation. Plasma samples were used for the analysis of glucose, total cholesterol and triacylglycerols by means of a dry-strip procedure (Spotchem, Menarini, Firenze, Italy), the levels of 3-hydroxybutyrate were measured with the kit 907979 from Boehringer Mannheim (Mannheim, Germany), and non-esterified fatty acids with the kit 1383175 from the same company. Insulin levels were measured through radioimmunoassay (rat insulin kit, Amersham, Amersham, United Kingdom). Results are shown in Table 2.

Analysis of the results shows that there were no globally significant changes (P<0.05, ANOVA) in the circulating levels of glucose, triacylglycerols, 3-hydroxybutyrate and non-esterified fatty acids because of the treatment with oleoyl-estrone. The drop in insulin levels, however, was significant, with lower values than controls from doses of 1 micromol/kg and day upwards. The decrease in insulin levels, albeit significant was less marked than in the rats of Example 1 affected by NIDDM. Glycaemia and blood lipids were maintained within a normal physiological range. The antidiabetic effect is the same as in Example 1, but the effects -dependent on the dose of oleoyl-estrone within a wide range of doses- were much less marked, i.e. the normality was not altered because the rats had levels already in the normal range.

### EXAMPLE 3: Effect of oleoyl-estrone on lipoproteins

Since treatment with oleoyl-estrone deeply modifies the plasma lipids of rats, an experiment was carried out to determine whether the oral administration of oleoyl-estrone modifies the plasma lipidogram of lean and genetically obese rats. Two groups of animals were used: (1) normal-weight adult female Zucker rats (genotype Fa/?), and (2) genetically obese adult female Zucker rats (genotype fa/fa). In the first group one rat was killed to obtain a sample of blood (intact control); in the second group (obese rats), two intact controls were killed and their blood recovered. All the remaining rats were kept under standard housing conditions, with free access to food and water; the rats receivid a daily gavage of 0,2 ml of sunflower oil by means of a stomach tube; in three rats form each group, the oil was supplemented with enough oleoyl-estrone to achieve a daily dose of 10 micromols per kg of rat weight. After 10 days of treatment, the rats were killed, and their blood was recovered. All blood samples were centrifuged to obtain plasma; plasma proteins were analysed by means of polyacrylamide gel electroforesis (kit Lipofilm, Sebia, Issy les Molineaux, France), lipids were stained with Sudan black.

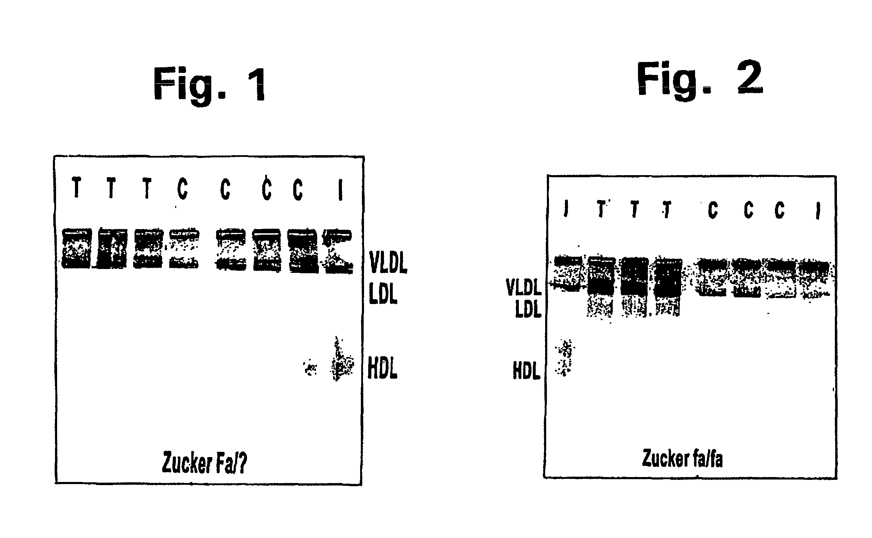

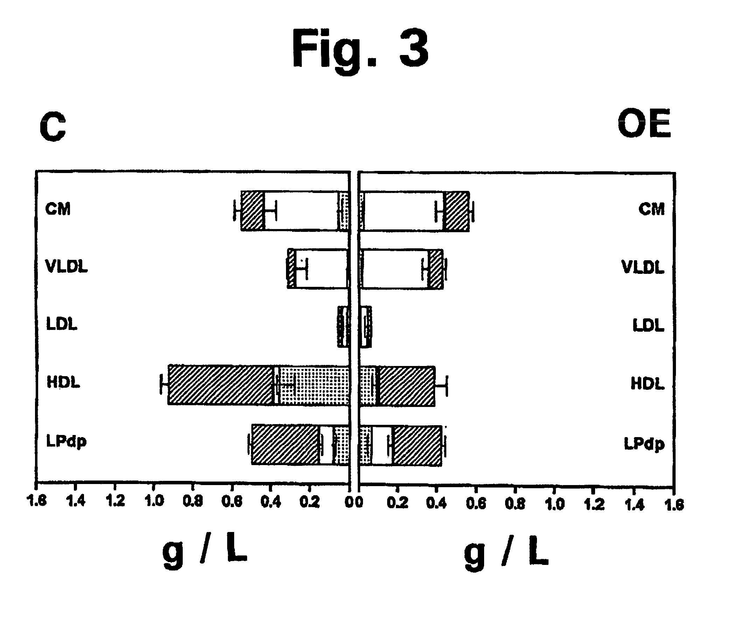

The results are presented in the accompanying figures: in Figure 1 the lipoproteinograms of normal-weight rat plasmas, and in Figure 2 those corresponding to obese rats. Intact rats (I) showed a normal lipoprotein pattern, with marked presence of LDL and, especially HDL. This same pattern can be observed in control rats (C), but in normal-weight animals treated with oleoyl-estrone (T) there was a marked reduction of high density lipoproteins, with a marked increase in VLDLs. In obese rats this same pattern is repeated, but there is an additional wide band between the LDL and VLDL zones. The lower lipid levels in LDL and HDL agrees with the fall in circulating cholesterol levels, and the increase in VLDL shows an active mobilisation of lipids. In all these results show the powerful hypolipemic effect of oleoyl-estrone, an effect that affects especially the levels and transport of cholesterol.

### EXAMPLE 4: Lipoprotein separation and lipid composition analysis

Lipoproteins in fresh plasma were fractionated by differential ultracentrifugation in known density media obtained through the addition of KBr. Five main fractions were obtained: chylomicra (CM) [density lower than 1.000 g/mL] very low density lipoproteins (VLDL) [density between 1.000 g/mL and 1.006 g/mL], low density lipoproteins (LDL) [density between 1.006 and 1.063 g/mL], high density lipoproteins (HDL) [density between 1.063 and 1.210 g/mL] and the 1.210 g/mL infranatant lipoprotein-depleted plasma (LPdp). These fractions were used for the analysis of protein and lipidic components, and for the estimation of particle size.

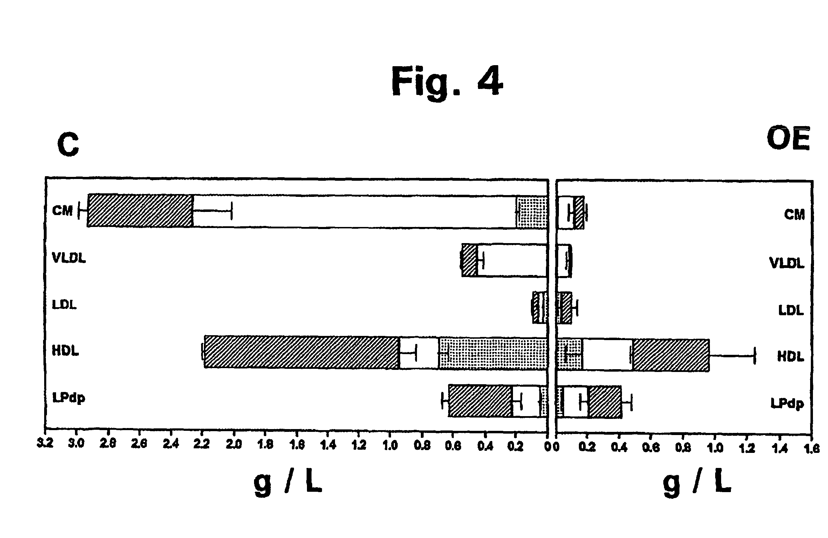

Whole plasma, lipoprotein fractions and LPdp were used for the analysis of protein and lipid content. Total protein was estimated with the Bradford method (cf. M.M. Bradford, Anal. Biochem. 1976, vol. 72, pp. 248-254). Total cholesterol was measured in saline with a high-performance cholesterol kit. Triacylglycerol concentrations were measured with a commercial kit and the results were corrected for free glycerol. Phospholipid was quantified by a choline-based method. The sum of total protein and lipid classes found in plasma fractions compared with the content of whole plasma (i.e. recovery) showed no differences between lean and obese rats. Recoveries for all plasma components (i.e. deviations from the values in whole plasma and those derived from the sum of its content in lipoprotein fractions) were lowest for triacylglycerols (88 ± 3 %) and highest for protein (92 ± 3 %). Sudan black-prestained serum samples were sepparated by polyacrylamide gel electrophoresis. Separation of lipoprotein classes through centrifugation allowed a detailed analysis of lipoprotein composition as shown in Figures 3 and 4.

In lean rats, oleoyl-estrone induced little change in lipoprotein composition, affecting essentially the HDL fraction, in which cholesterol decreased most (which was reflected in the lower whole plasma cholesterol levels of lean treated rats), followed by phospholipid.

Control obese rats showed higher triacylglycerol, phospholipid and cholesterol levels than lean controls, an effect fully observed in total plasma and chylomicra. VLDLs also presented higher triacylglycerols and phospholipids, LDLs had higher cholesterol, and HDL showed higher phospholipids and cholesterol in obese than in lean controls.
Treatment with oleoyl-estrone induced marked decreases in all chylomicra and VLDL components of obese rats; in LDLs only tricylglycerol levels decreased and in HDLs all components decreased again with treatment, except for triacylglycerols. This resulted in markedly lower levels of all lipid classes in whole plasma of obese treated rats compared with their controls.

When both oleoyl-estrone-treated groups, lean and obese were compared, in chylomicra and VLDL, both protein and triacylglycerols decreased, as did the VLDL phospholipids. In contrast, HDL triacylglycerol levels in treated obese rats were higher than in the lean, and no differences were observed at all in LDLs.

Lipoprotein-depleted plasma composition did not differ significantly because of obesity; oleoyl-estrone treatment only induced a decrease in total phospholipid levels in both lean and obese animals.

### EXAMPLE 5: Measurement of the activity of lipases

Liver, muscle and WAT (white adipose tissue) samples were homogenized in 10 mM hepes buffer pH 7.5, containing 250 mM sucrose, 1 mM-EDTA and 1 mM dithiothreitol in order to measure lipoprotein lipase activity in tissues. Homogenates were clarified by centrifugation (10 min at 10,000xg) at 4 °C. The assay mixture (pH 7.5) contained 0 . 6 mM tri-[9,10(n)-³H]-oleoyl-glycerol, 50 mM MgCl₂, 0.5 g/L albumin (fatty acid-free), 3 ml/L preheated (60 min at 50° C)rat serum, 25 mM PIPES buffer and 0.02 ml of sample in a final volume of 0.2 ml; the incubation was carried out for 30 min at 25 °C; after the reaction was stopped, the [³H]-oleate evolved was quantified . All measurements of lipoprotein lipase activity in plasma and liver homogenates were carried out in the presence of anti-rat hepatic lipase antiserum. The homogenate and plasma protein content were determined with the Bradford method.

The enzyme activity was expressed in nkat/L of plasma of nkat/kg of tissue; the values were also referred to plasma or tissue protein content. The overall share of potential activity of the enzymes studied was also estimated by determining the contribution of each organ/tissue: plasma, liver and WAT (white adipose tissue) enzyme activity in relation to body mass. The results are expressed as the total activity found per kg of body weight in a given tissue or compartment. The liver was directly weighed (3.38 ± 0.10 % of BW (body weight) for lean and 3.17 ± 0.13 % of BW for the obese). The mass of plasma was taken as a 2.75 % of body weight (blood being 5 % of body weight, and packed cell volume 45 % of that value). Overall WAT lipoprotein lipase activity in both groups of animals was calculated using the mean values for either the periovaric or mesenteric locations of both sets of data and the proportions of WAT published for lean [15 % of BW] control and oleoyl-estrone-treated [7 % of BW] and obese Zucker fa/fa rat [35 % BW] control and oleoyl-estrone-treated [28 % BW]. Muscle mass was calculated also as a proportion of body weight: 43 % for lean and 33 for obese rats.

Statistical analysis of the differences between groups was established using ANOVA programs and the Student's t test.

Table 3 shows the lipoprotein lipase activity on plasma, liver, WAT and muscle of lean and obese rats under treatment with oleoyl-estrone compared with controls. In lean rats, oleoyl-estrone treatment did not affect significantly the lipoprotein lipase activity of the tissues studied, with the exception of a generalized rise in plasma.

Obese controls showed higher lipoprotein lipase activity in WAT compared with their lean counterparts. Oleoyl-estrone-treated obese rats, however, showed decreases in WAT, no changes in liver and increases in plasma, and specially in muscle. We estimated roughly the overall lipoprotein lipase activity in the rat simply by adding the activities contributed by muscle, liver and plasma, plus that of WAT, calculated as the mean of mesenteric and periovaric activities per g of tissue and applied to the estimated mass of body WAT. Lean rats showed a marked decrease in overall lipoprotein lipase activity: from about 240 nkat in control rats to 152 nkat in treated rats; in the obese, the decrease was similar: from 684 nkat per control rat to 338 nkat in treated rats. The overall loss of lipoprotein lipase because of oleoyl-estrone treatment was 37% and 51 %, respectively, in lean and obese animals. This loss was maily the consequence of a decrease in WAT lipoprotein lipase, which was more marked in mesenteric WAT (down by 64% -lean- and 87% - obese-) than in periovaric WAT (down by 45 % -lean- and 56% -obese-). In contrast, the lipoprotein lipase activity in skeletal muscle of obese rats increased fourfold, which partly compensated the loss of WAT lipoprotein lipase; in these animals, the contribution of muscle lipoprotein lipase changed from about 1/8 or1/25 of that of WAT (depending on which location of WAT we compare the muscle figure) in controls to roughly the same activity in both WAT and muscle in oleoyl-estrone-treated rats.

**Table 1:**

| Effect of the oral administration of oleoyl-estrone for 10 days in daily gavages on the plasma parameter of Zucker fa/fa female and male rats. | | | | | |
|---|---|---|---|---|---|
| plasma parameter | unit | female obese rats | | male obese rats | |
| | | 0 µmol/kg·d | 10 mol/kg·d | 0 mol/kg·d | 10 µmol/kg·d |
| glucose | mM | 6.21 ±0.16 | 4.91 ±0.17 | 7.42 ±0.44 | 6.14 ±0.13 |
| insulin | nM | 4.54 ±0.49 | 1.78 ±0.21 | 10.65 ±1.85 | 1.11 ±0.12 |
| triacylglycerols | mM | 3.59 ±0.06 | 1.80 ±0.17 | 3.15 ±0.21 | 1.88 ±0.19 |
| 3-hydroxybutyrate | µM | 225 ±15 | 187 ±26 | 283 ±33 | 281 ±31 |
| non-esterified fatty acids | µM | 682 ±56 | 642 ±52 | 500 ±13 | 720 ±110 |
| total cholesterol | mM | 2.29 ±0.22 | 1.59 ±0.29 | 2.71 ±0.04 | 1.77 ±0.26 |
| The data are the mean ± SEM of 6 animals per group. | | | | | |

**Table 2 -**

| Effect of different doses of oleoyl-estrone administration for 10 days in daily gavages on the plasma parameters of female Wistar rats. Dose = daily dose of oleoyl-estrone in µmol/kg·d | | | | | | | | | |
|---|---|---|---|---|---|---|---|---|---|
| plasma parameter | unit | Dose | | | | | | | |
| | | 0 | 0.2 | 0.5 | 1 | 2 | 5 | 10 | 20 |
| glucose | mM | 6.42 ±0.33 | 6.35 ±0.47 | 5.74 ±0.31 | 5.71 ±0.46 | 5.76 ±0.20 | 5.64 ±0.21 | 5.90 ±0.31 | 5.62 ±0.20 |
| Insulin | nM | 0.337 ±0.067 | 0.624 ±0.138 | 0.497 ±0.067 | 0.241 ±0.044 | 0.280 ±0.036 | 0.275 ±0.053 | 0.262 ±0.050 | 0.223 ±0.041 |
| triacylglycerols | mM | 1.58 ±0.30 | 1.57 ±0.43 | 1.79 ±0.23 | 1.26 ±0.26 | 1.70 ±0.11 | 1.91 ±0.33 | 1.39 ±0.14 | 0.92 ±0.11 |
| 3-hydroxybutyrate | µM | 237 ±32 | 291 ±40 | 189 ±41 | 305 ±56 | 263 ±27 | 300 ±64 | 301 ±42 | 237 ±47 |
| non-esterified fatty acids | µM | 221 ±45 | 184 ±31 | 273 ±47 | 147 ±30 | 281 ±44 | 213 ±48 | 152 ±24 | 122 ±39 |
| total cholesterol | mM | 1.14 ±0.10 | 1.21 ±0.12 | 1.02 ±0.08 | 1.13 ±0.15 | 1.01 ±0.27 | 1.03 ±0.13 | 0.66 ±0.09 | 0.53 ±0.05 |

The data are the mean ± SEM of 6 animals per group

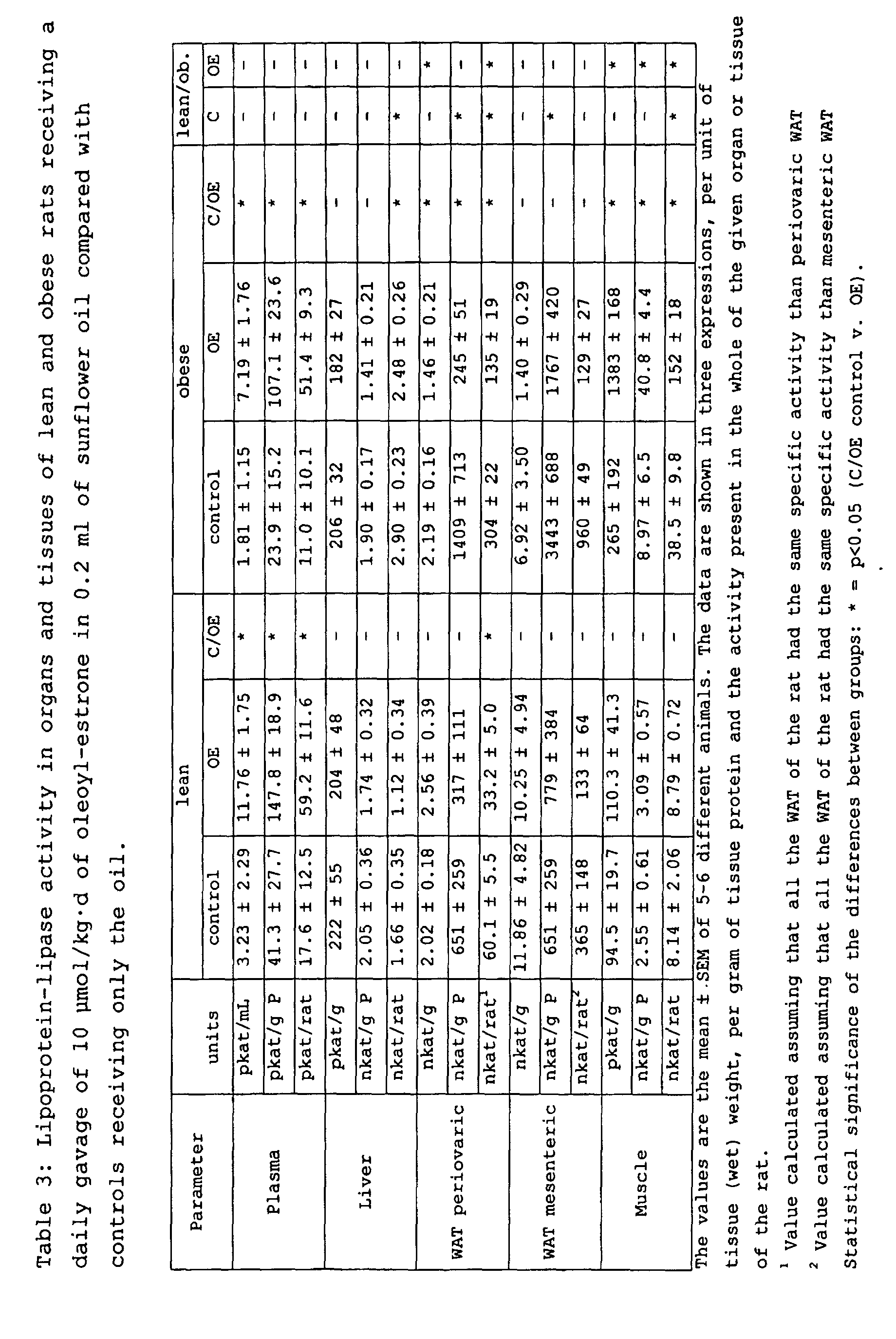

## Claims

1. Use of oleoyl-estrone for the preparation of a medicament for the treatment and/or profilaxis of a human suffering from a metabolic disease selected from the group consisting of diabetes mellitus type 2, hyperlipidemia, and the Metabolic Syndrome X associated with glucose intolerance, hyperinsulinemia, or insulin resistance.

2. Use according to claim 1, wherein the metabolic disease is diabetes mellitus type 2 (non-insulin-dependent).

3. Use according to claim 1, wherein the metabolic disease is hyperlipidemia.

4. Use according to claim 3, wherein the hyperlipidemia is hypertriacylglyceridemia.

5. Use according to claim 3, wherein the hyperlipidemia is hypercholesterolemia.

6. Use according to claim 3, wherein the hyperlipidemia is hyperlipoproteinemia.

7. Use according to claim 1, wherein the metabolic diasease is Metabolic Syndrome X associated with glucose intolerance, hyperlipidemia, hyperinsulinemia, or insulin resistance.

8. Use according to any of the preceding claims, where the medicament is for oral administration.

9. Oleoyl-estrone for the treatment and/or profilaxis of a human suffering from a metabolic disease selected from the group consisting of diabetes mellitus type 2, hyperlipidemia, and the Metabolic Syndrome X associated with glucose intolerance, hyperinsulinemia, or insulin resistance.

10. Oleoyl-estrone according to claim 9, wherein the metabolic disease is diabetes mellitus type 2 (non-insulin-dependent).

11. Oleoyl-estrone according to claim 9, wherein the metabolic disease is hyperlipidemia.

12. Oleoyl-estrone according to claim 11, wherein the hyperlipidemia is hypertriacylglyceridemia.

13. Oleoyl-estrone according to claim 11, wherein the hyperlipidemia is hypercholesterolemia.

14. Oleoyl-estrone according to claim 11, wherein the hyperlipidemia is hyperlipoproteinemia.

15. Oleoyl-estrone according to claim 9, wherein the metabolic disease is Metabolic Syndrome X associated with glucose intolerance, hyperlipidemia, hyperinsulinemia, or insulin resistance.

16. Oleoyl-estrone according to any of the claims 9-15, for oral administration.
